(19) Europäisches Patentamt
European Patent Office
Office européen des brevets

(11) Veröffentlichungsnummer: **0 001 271**
**B1**

(12) # EUROPÄISCHE PATENTSCHRIFT

(45) Veröffentlichungstag der Patentschrift: **27.10.82**

(21) Anmeldenummer: **78100908.9**

(22) Anmeldetag: **15.09.78**

(51) Int. Cl.³: **C 07 D 205/12,** C 07 D 229/00, C 07 D 487/08, C 07 D 491/08, C 07 D 498/08, A 01 N 43/90 //(C07D487/08, 203/00, 205/00, 229/00, 231/00, 249/00), (C07D491/08, 205/00, 229/00, 303/00), (C07D498/08, 205/00, 229/00, 261/00)

(54) **Heterocyclische Norbornanderivate, Mittel zur Beeinflussung des Pflanzenwachstums die diese Verbindungen enthalten, und Verfahren zur Herstellung dieser Mittel.**

(30) Priorität: **19.09.77 DE 2742034**

(43) Veröffentlichungstag der Anmeldung:
**04.04.79 Patentblatt 79/7**

(45) Bekanntmachung des Hinweises auf die Patenterteilung:
**27.10.82 Patentblatt 82/43**

(84) Benannte Vertragsstaaten:
**BE CH DE FR GB LU NL SE**

(56) Entgegenhaltungen:
**DE - A - 2 615 878**

(73) Patentinhaber: **BASF Aktiengesellschaft**
**Carl-Bosch-Strasse 38**
**D-6700 Ludwigshafen (DE)**

(72) Erfinder: **Platz, Rolf, Dr.**
**Hansastrasse 5**
**D-6800 Mannheim 1 (DE)**
Erfinder: **Fuchs, Werner, Dr.**
**Muenchbuschweg 30a**
**D-6700 Ludwigshafen (DE)**
Erfinder: **Rieber, Norbert, Dr.**
**Wilhelmstrasse 77**
**D-6800 Mannheim 51 (DE)**
Erfinder: **Jung, Johann, Dr.**
**Hardenburgstrasse 19**
**D-6703 Limburgerhof (DE)**
Erfinder: **Wuerzer, Bruno, Dr.**
**Wilhelm-Busch-Strasse 55**
**D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 271 B1

# Heterocyclische Norbornanderivate, Mittel zur Beeinflussung des Pflanzenwachstums, die diese Verbindungen enthalten, und Verfahren zur Herstellung dieser Mittel

Die vorliegende Erfindung betrifft neue wertvolle polycyclische Verbindungen, Verfahren zur ihrer Herstellung, Pflanzenschutzmittel, die diese Verbindung enthalten, und ihre Verwendung als Pflanzenschutzmittel.

Es wurde gefunden, daß Verbindungen der Formel

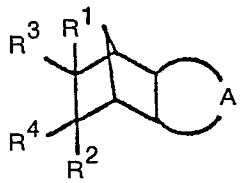

in der A die Reste 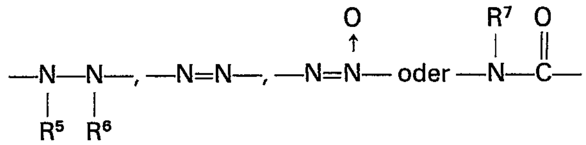

bedeutet, wobei $R^5$ und $R^6$ je ein Wasserstoffatom oder einen Alkoxycarbonylrest und $R^7$ ein Wasserstoffatom oder die Gruppe $-SO_2Cl$ bedeutet und $R^1$ und $R^2$ entweder zusammen die Reste

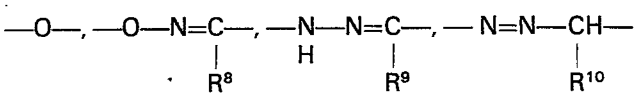 und, wenn A die Reste

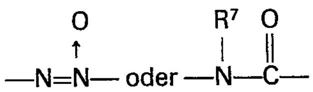 bedeutet, auch die Reste 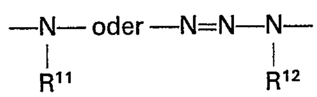

bedeuten, wobei die Reste

$R^8$ bis $R^{12}$ je einen Phenylrest, der durch Halogen, Nitro, Alkoxy, prim., sec. oder tert. Alkyl oder Halogenalkyl substituiert sein kann oder einen 2-Thiazolyrest bedeutet oder

$R^1$ und $R^2$ als einzelne Reste entweder je ein Halogenatom oder einer dieser Reste Halogen, OH oder Alkoxycarbonyl und der andere einen Rest der Formel, $R^{12}NH-$ bedeutet und $R^3$ und $R^4$ je ein Wasserstoffatom bedeuten, wobei einer dieser Reste auch ein Halogenatom bedeuten kann, wenn $R^1$ und $R^2$ zusammen den Rest —O— bedeuten, eine gute herbizide und wachstumsregulierende Wirkung haben, die besser als die entsprechende Wirkung bekannter Wirkstoffe ist.

Es ist bekannt, die Verbindungen III—V, mit $R{=}CH_3-$ und $C_2H_5-$, auf folgendem Syntheseweg herzustellen: Reaktion von Quadricyclan (I) mit Azodicarbonsäurediestern (II) zu III, das durch Verseifung und Decarboxylierung in IV umgewandelt und ohne Isolierung von IV durch Oxidation mit $CuCl_2$ über einen $Cu^I$-Komplex in die Azoverbindung V überführt wird (JACS *91*, 5668 (1969)).

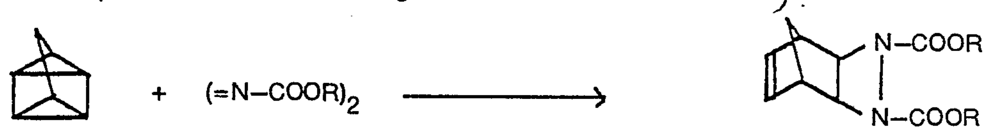


I II III

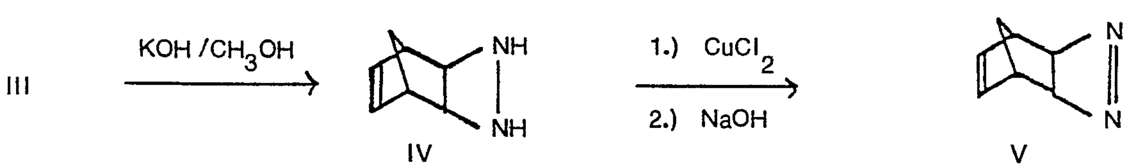


IV V

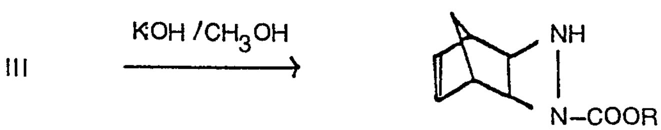


VI

Man erhält die Verbindung VI durch partielle Verseifung und Decarboxylierung von III. Außerdem ist die Verbindung VII bekannt (J. Org. Chem. *33*, 370 (1968)).

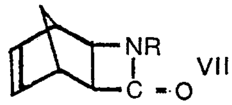
VII

R = H, $-SO_2Cl$

Aus diesen bekannten Verbindungen lassen sich die erfindungsgemäßen Wirkstoffe wie folgt herstellen:

1. Herstellung der in den Beispielen 1—3 und den zugehörigen Tabellen beschriebenen Verbindungen. Umsetzung der Verbindungen III—VII mit 1,3-dipolaren Agentien.

Die vielfach beschriebene (Houben-Weyl, Bd. 5/Ib, S. 1119 ff. und dort zitierte Literatur) Umsetzung des unsubstituierten Norbornens mit 1,3-dipolaren Agentien zu polycyclischen Fünfring-Systemen läßt sich auch bei den Norbornen-Derivaten III—VII durchführen. Auf diesem Wege lassen sich durch Addition von stabilen 1,3-dipolaren Agentien, z.B. Diazoverbindungen und Aziden, oder von 1,3-dipolaren Agentien, die in bekannter Weise in situ hergestellt werden, z.B. Nitriloxide, die entsprechenden $\Delta^1$- oder $\Delta^2$-Pyrazolin-, Triazolin- und Isoxazolin-Derivate der Verbindungen III—VII herstellen.

In einigen Fällen bilden sich unter den Reaktionsbedingungen aus den primär entstehenden Triazolinen durch $N_2$-Eliminierung die entsprechenden Aziridin-Derivate.

Bei den stabilen Triazolinen erreicht man die $N_2$-Eliminierung durch Erhitzen in inerten Lösungsmitteln auf 80—140°C oder durch Zusatz von Säuren, z.B. Trifluoressigsäure, Essigsäure oder Schwefelsäure, bei 20—80°C (Beispiel 3 B.).

2. Herstellung der im Beispiel 4 A. und der dazugehörigen Tabelle beschriebenen Verbindungen. Ringöffnung der Triazolin- oder Aziridin-Derivate von III—VII mit Säuren.

Die Triazolin- oder Aziridin-Derivate der Verbindungen III—VII können mit Halogenwasserstoffsäuren in Wasser oder inerten Lösungsmitteln, wäßriger Schwefelsäure oder Carbonsäuren in die entsprechenden 1,2-Aminohalogen-, 1,2-Amino-hydroxy- oder 1,2-Amino-acyloxy-Derivate überführt werden. Die Umsetzung erfolgt leicht durch Mischen der Komponenten in Wasser bzw. inerten Lösungsmitteln bei —20 bis +30°C oder durch Erhitzen des Reaktionsgemisches auf 40—130°C.

3. Herstellung der im Beispiel 4 B. und der dazugehörigen Tabelle beschriebenen Verbindungen. Umsetzung der Verbindungen III—VII mit Halogenen bzw. Halogen-Verbindungen.

Durch Reaktion der Kohlenstoff-Doppelbindung der Norbornen-Derivate III—VII mit Halogenen bzw. Halogenverbindungen lassen sich zahlreiche Halogen-Verbindungen synthetisieren. Diese Reaktionen sind mit acyclischen und cyclischen Olefinen (z.B. Norbornen) vielfach beschrieben (Houben-Weyl, Bd. 5/1b, S. 981 ff, Bd. 5/3, S. 72, 95, 529, 762, 813 ff, Bd. 5/4, S. 38, 533 ff, Bd. 10/1, S. 61 ff; Synthesis *1977*, 462 ff) und können auch auf die Derivate III—VII übertragen werden. So erhält man durch Umsetzung mit Halogenen, Interhalogenverbindungen oder Hypohalogeniten, die entsprechenden Additionsverbindungen.

Aus einigen Additionsprodukten von III oder VII ($R = SO_2Cl$) lassen sich analog der Umwandlung III→ V oder VII ($R = SO_2Cl$)→ VII ($R = H$) auch die entsprechenden Additionsprodukte von V oder VII ($R = H$) herstellen. Dabei entstehen z.T. durch Halogenwasserstoffabspaltung (Rückbildung der Kohlenstoff-Doppelbindung) auch die monosubstituierten Norbornen-Derivate, die zur Herstellung der entsprechenden Oxiran- Derivate gemäß Beispiel 5 eingesetzt werden können.

4. Herstellung der im Beispiel 5 und der dazugehörigen Tabelle beschreibenen Verbindungen. Oxidation der Verbindungen III—VII oder daraus gemäß 1., 2. und 3. hergestellter Derivate.

Die Kohlenstoff-Doppelbindung der Norbornen-Derivate III bis VII und/oder die Stickstoff-Doppelbindung des Norbornen-Derivates V bzw, der Derivate von V, die nach den oben (1., 2. und 3.) beschriebenen Methoden hergestellt wurden, können nach bekannter Weise oxidiert werden. Dabei entstehen durch Umsetzung mit organischen Persäuren (Houben-Weyl, Bd. 6/3, S. 385 ff, Bd. 10/2, S. 787 ff) die entsprechenden Oxiran und/oder Azoxy-Derivate.

Die Oxiran-Derivate von V lassen sich aus den entsprechenden Derivaten von III analog der Umwandlung III→V herstellen.

Die Struktur der Wirkstoffe wurde durch NMR-, IR-, Massenspektroskopie oder Elementaranalyse belegt.

Die angegebenen Schmelz- oder Zersetzungspunkte sind nicht korrigiert.

Beispiel 1

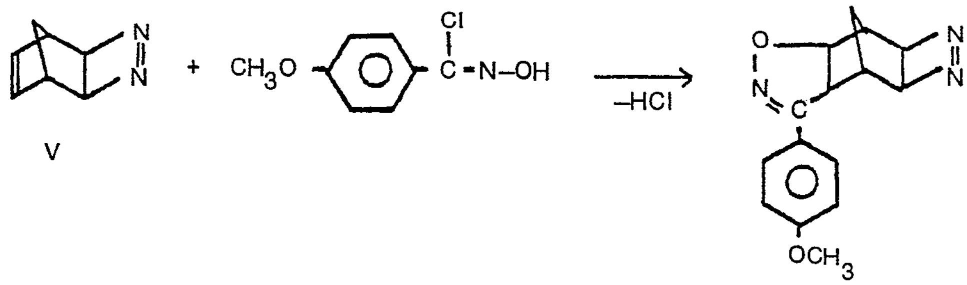


V

Zu 20,6 Teilen V, 19,6 Teilen Triäthylamin und 400 Teilen Äther fügt man 32 Teile p-Methoxy-Chlorbenzaldoxim und rührt 12 Stunden bei 25°C. Das Festprodukt wird abgesaugt, mit 100 Teilen Wasser gewaschen und getrocknet.
Ausbeute: 25,2 Teile (55% der Theorie).
Fp. 160°C Zers. (Toluol/Ligroin).

Entsprechend Beispiel 1 wurden folgende Verbindungen hergestellt:

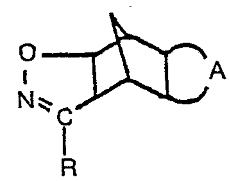

| R | A | Fp. [°C] |
|---|---|---|
| $C_6H_5-$ | –N=N– | 173 |
| p-Br–$C_6H_4$– | ,, | 196 |
| ,, | –N(H)–N($COOCH_3$)– | 141 * |
| p-Cl–$C_6H_4$– | –N=N– | 185 (Z) |
| p-Cl–$C_6H_4$– | –N(H)–C(=O)– | 214 (Z) |
| p-$CH_3$–$C_6H_4$– | –N=N– | 181 (Z) |
| p-$NO_2$–$C_6H_4$– | ,, | 212 (Z) |
| ,, | –N(H)–N($COOCH_3$)– | 167 * |
| ,, | –N($COOCH_3$)–N($COOCH_3$)– | 169 |
| 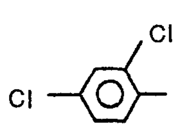 | –N(H)–C(=O)– | 161 * |

* Stellungsisomere

Beispiel 2

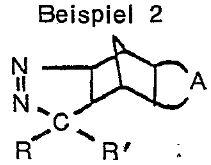

| R′ | R | A | Fp. °C |
|---|---|---|---|
| $C_6H_5-$ | H– | –N=N– | 172 (Z) |
| p-Cl–$C_6H_4-$ | H– | –N=N– | 149 (Z) |
| ,, | H– | –N——N–<br>COOCH$_3$ H | 90 * |
| p-CH$_3$–$C_6H_4-$ | H– | –N——N–<br>COOCH$_3$ H | 91 * |
| ,, | H– | H O<br>–N–C– | 230 (Z) * |
| p-Cl–$C_6H_4$ | H– | –N——N–<br>COOCH$_3$ COOCH$_3$ | 156 (Z) |
| m-NO$_2$–$C_6H_4-$ | H– | –N=N– | |

* Stellungsisomere

Beispiel 3

A. 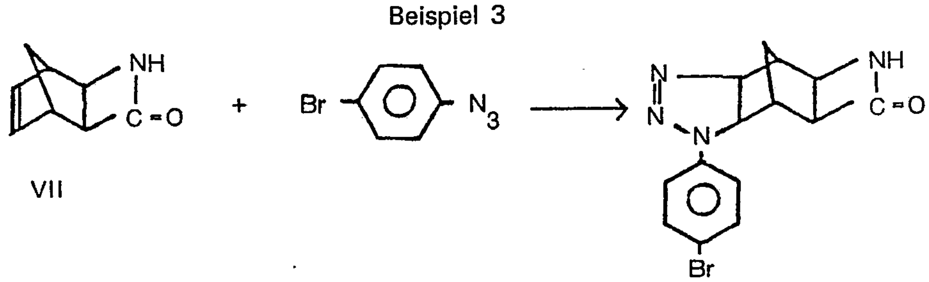

VII

5 Teile VII und 3,4 Teile p-Bromphenylazid werden 6 Stunden in 40 Teilen Toluol bei 80°C gerührt. Nach dem Abkühlen wird das entstandene Triazolinderivat (Stellungsisomere) abgesaugt und mit 50 Teilen Petroläther gewaschen.
Ausbeute: 7,4 Teile (88% der Theorie).
Fp 208°C (Zers.).

B. 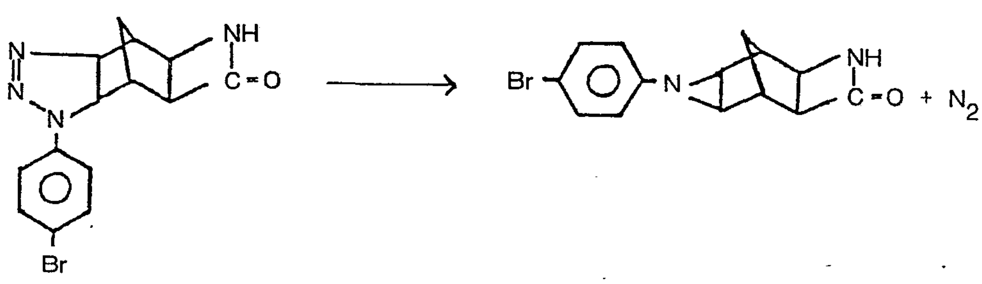

6,8 Teile des oben angeführten Triazolin-Derivates (Isomerengemisch) werden in je 25 Teilen Essigsäure und Äther bei 25°C 4 Stunden gerührt. Danach wird das Aziridin-Derivat abgesaugt und mit 20 Teilen Äther gewaschen.
Ausbeute: 6,1 Teile (90% der Theorie).
Fp. 228°C.

Entsprechend Beispiel 3 wurden folgende Verbindungen hergestellt:

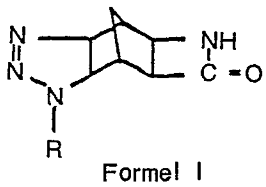

Formel I

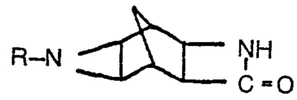

Formel II

| Formel | R | FP. [°C] |
|---|---|---|
| I | $C_6H_5-$ | 200 (Z) * |
| II | ,, | |
| I | m-$CF_3-C_6H_4-$ | |
| I | 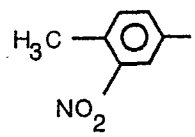 | 174 (Z) * |
| ,, | 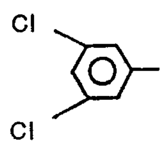 | 216 (Z) * |
| I | 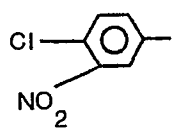 | 195 * |
| I | 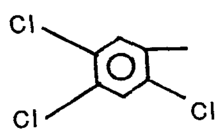 | 115 (Z) * |
| II | ,, | |
| I | 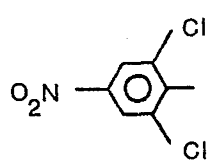 | 141 (Z) * |
| I | p-F-$C_6H_4-$ | 185 (Z) * |
| ,, | 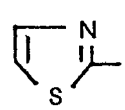 | 153 (Z) * |
| II | ,, | 176 * |

* Stellungsisomere

Beispiel 4

A. 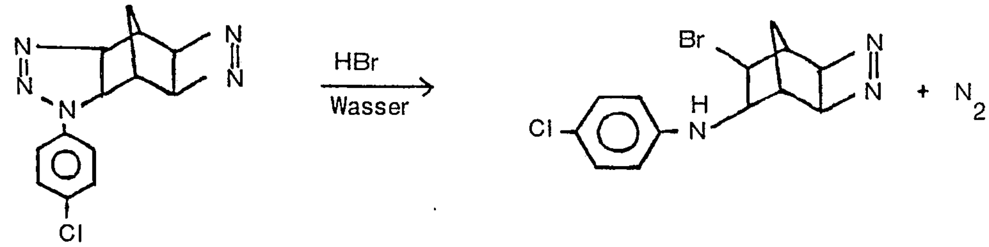


5,4 Teile des N-p-Chlorphenyl-Δ¹-1,2,3-Triazolin-Derivates werden in 7 Teilen 47 proz. wäßriger HBr und 50 Teilen Wasser 5 Stunden auf 100°C erhitzt. Nach dem Abkühlen wird das Festprodukt abgesaugt und mit 200 Teilen Wasser gewaschen.
Ausbeute: 5,7 Teile (88,5% der Theorie).
Fp. 185°C (Zers.)

B. 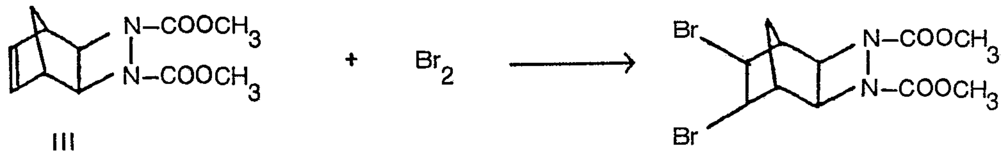

III

Zu 15 Teilen III und 50 Teilen $CH_2Cl_2$ tropft man bei –10°C 10,1 Teile Brom in 25 Teilen $CH_2Cl_2$. Die Reaktionslösung wird 1 Stunde bei –10°C nachgerührt, anschließend mit 0,5 Teilen Aktivkohle behandelt, abfiltriert und bei 25°C und 15 Torr eingeengt. Der Rückstand wird mit 40 Teilen Petroläther gewaschen.
Ausbeute 20 Teile (80% der Theorie).
FP 103°C.

Ähnlich Beispiel 4 wurden folgende Verbindungen hergestellt:

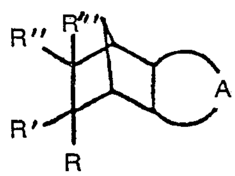

| R | R′ | R″ | R‴ | A | Fp. [°C] |
|---|---|---|---|---|---|
| Cl– | H– | Cl– | H– | –N=N– | 93 |
| " | " | " | " | H O<br>‖<br>–N–C– | 55 * |
| " | " | " | " | –N——N–<br>$COOCH_3$ $COOCH_3$ | 45 |
| Br– | " | Br– | " | –N——N–<br>$COOC_2H_5$ $COOC_2H_5$ | 16 |
| " | " | " | " | –N=N– | 83 |
| " | " | HO– | " | –N——N–<br>$COOCH_3$ $COOCH_3$ | |
| " | " | " | " | –N=N– | |

* Stellungsisomere

| R | R′ | R″ | R‴ | A | Fp. [°C] |
|---|---|---|---|---|---|
| Cl | H– | Cl– | H– | 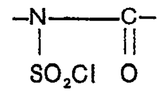 | 35 * |
| Br– | ,, | Br– | ,, | 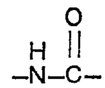 | 141 * |
| Br– | ,, | Br– | ,, | 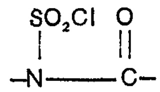 | 5 * |
| Br– | ,, | Br– | ,, | 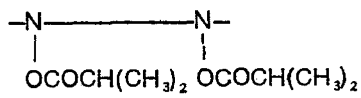 | 21 |
| 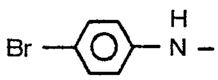 | ,, | Cl– | ,, | –N=N– | 193 (Z) |
| ,, | ,, | J– | ,, | ,, | 183 (Z) |
| ,, | ,, | HO– | ,, | ,, | 214 (Z) |
| 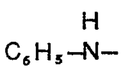 | ,, | Br– | ,, | ,, | |
| ,, | ,, | J– | ,, | ,, | |
| 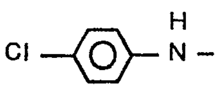 | ,, | Cl– | ,, | –N=N– | 158 (Z) |
| ,, | ,, | F– | ,, | ,, | |
| ,, | ,, | Br– | ,, | 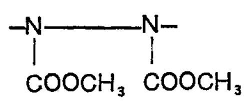 | 110 |
| 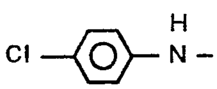 | ,, | HO– | ,, | –N=N– | 216 (Z) |
| 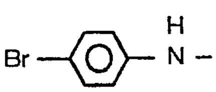 | ,, | $CH_3COO$– | ,, | –N=N– | 166 (Z) |
| ,, | ,, | ,, | ,, | 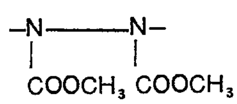 | |

* Stellungsisomere

| R | R' | R'' | R''' | A | Fp. [°C] |
|---|---|---|---|---|---|
| 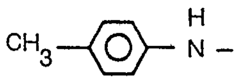 | H– | Br– | H– | –N=N– | 161 (Z) |
| „ | „ | Cl– | „ | „ | 143 (Z) |
| 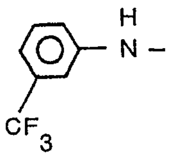 | „ | Br– | „ | –N=N– | 148 (Z) |
| „ | „ | J– | „ | „ | 146 (Z) |
| 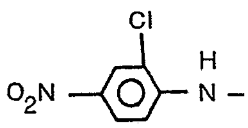 | „ | Br– | „ | –N–––N– $COOCH_3$ $COOCH_3$ | 239 (Z) |
| 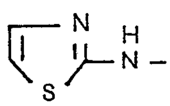 | „ | Br– | „ | –N=N– | 151 (Z) |
| „ | „ | „ | „ | –N–––N– $COOCH_3$ $COOCH_3$ | |
| 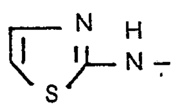 | H– | Cl– | H– | –N=N– | 153 (Z) |

Beispiel 5

A. 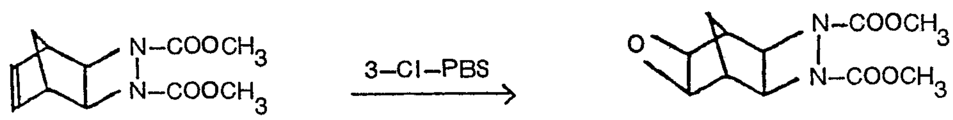


III

30 Teile III und 43,5 Teile 3-Chlorperbenzoesäure (3-Cl-PBS, 70-prozentig) werden 6 Stunden in 800 Teilen 1,1,2-Trichloräthan bei 70°C gerührt. Nach dem Abkühlen wird die Lösung filtriert und mit je 250 Teilen gesättigter wäßriger KJ-, $Na_2S_2O_3$- und $NaHCO_3$-Lösung extrahiert. Die organische Phase wird mit $MgSO_4$ getrocknet und bei 25°C und 15 Torr eingeengt. Der Rückstand wird mit 100 Teilen Petroläther gewaschen.
Ausbeute 27,8 Teile (87% der Theorie).
FP 130°C (Benzol/Ligroin).

B. 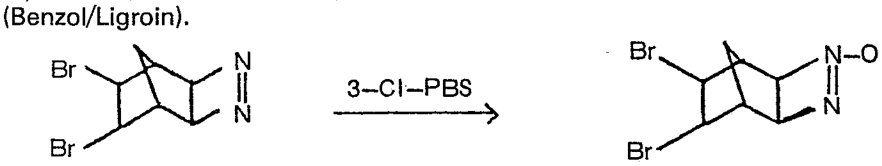


Stellungsisomere

1,5 Teile des Dibrom-Derivates von V und 2,7 Teile 3-Chlorperbenzoesäure (70 %ig) werden 2 Stunden in 20 Teilen 1,1,2-Trichloräthan bei 70°C gerührt. Nach dem Abkühlen wird die Lösung filtriert und mit je 30 Teilen gesättigter wäßriger KJ-, $Na_2S_2O_3$- und $NaHCO_3$-Lösung extrahiert. Die or-

ganische Phase wird mit $MgSO_4$ getrocknet und bei 25°C und 15 Torr eingeengt. Der Rückstand wird mit 10 Teilen Petroläther gewaschen.
Ausbeute 1,2 Teile (78% der Theorie).
Fp 116°C.

Ahnlich Beispiel 5 wurden folgende Verbindungen hergestellt:

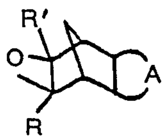

Formel I

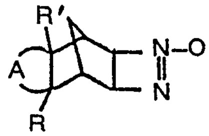

Formel II

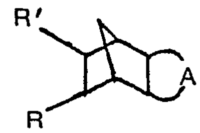

Formel III

| Formel | R | R' | A | Fp. [°C] |
|---|---|---|---|---|
| I | H– | H– | –N=N– | 169 (Z) |
| I | H | H | 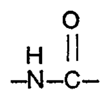 | |
| I | H | H | 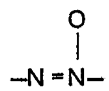 | 171 |
| III | Cl– | Cl– | 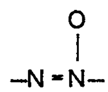 | * |
| I | H– | H– | 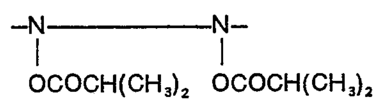 | 52 |
| II | Br– | H | –O– | 20 * |
| ,, | Cl– | ,, | ,, | * |
| II | H– | H– | 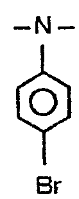 | 235 (Z) |
| II | H | H | 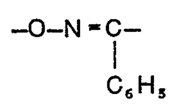 | 198 (Z) * |
| ,, | ,, | ,, | 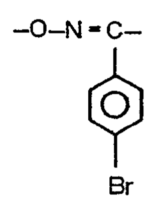 | 196 (Z) * |

* Stellungsisomere

Beispiel 6

Herstellung von Ausgangsverbindungen (nicht erfindungsgemäß)

A. 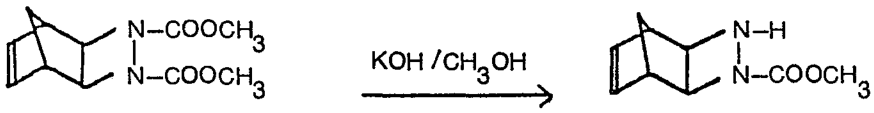


III IV

Zu 60 Teilen der Verbindung III und 50 Teilen Methanol tropft man unter Rühren bei 65°C eine Lösung von 35 Teilen KOH in 60 Teilen Methanol. Das Reaktionsgemisch wird anschließend noch 2 Stunden bei 65°C gerührt. Danach wird das Lösungsmittel bei 50 mbar abdestilliert und der Rückstand mehrmals mit insgesamt 1 200 Teilen Äther extrahiert. Nach Abdestillieren des Äthers erhält man 38 Teile der Verbindung IV (85% der Theorie, Fp 94°C aus Ligroin).

B. 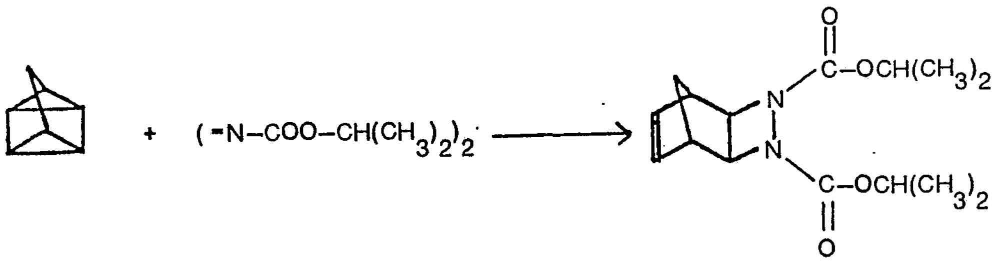


Zu 20 Teilen Quadricyclan in 100 Teilen Benzol tropft man bei 80°C 40,5 Teile Azodicarbonsäure-di-isopropylester. Danach wird das Gemisch noch 24 Stunden auf 80°C erhitzt und anschließend das Lösungsmittel bei 50 mbar abgezogen.

Ausbeute: 58 Teile (98% der Theorie).

Fp 41°C.

Die im Beispiel 6 aufgeführten Verbindungen können als Ausgangsprodukte für Wirkstoff-Synthesen eingesetzt werden (vgl. Tabellen in den Beispielen 1, 2, 4 und 5).

Die neuen Wirkstoffe haben eine starke biologische Wirkung auf Pflanzen, d.h. sie beeinflussen das Pflanzenwachstum, sei es als Hemmung des Längenwachstums, sei es als Veränderung der Konzentration der Pflanzeninhaltsstoffe, sei es als Vernichtung unerwünschter Pflanzen bei gleichzeitiger Schonung der Nutzpflanzen.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle use., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Athanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon und Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxylierte Isooctylphenol-, Octylphenol-, Nonylphenol-, Alkylphenol-polyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes

Polyoxypropylen, Laurylalkoholpolyglykolätheracetal, Sorbitester, Lignin, Sulfitablaugen und Methylcellulose.

Pulver, Streu- und Staubmittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kalk, Kreide, Talkum, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen erhalten zwischen 0,1 und 95 Gewichtsprozent Wirkstoff, vorzugsweise zwischen 0,5 und 90 Gewichtsprozent.

Zu den Mischungen oder Einzelwirkstoffen können Öl verschiedenen Typs, Herbizide, Fungizide, Nematozide, Insektizide, Bakterizide, Spurenelemente, Düngemittel, Antischaummittel (z.B. Silikone) oder Wachstumsregulatoren zugemischt werden.

In verschiedenen Fällen kann sich die Kombination bzw. Mischung der erfindungsgemäßen Substanzen mit anderen wachstumsregulierenden Wirkstoffen als vorteilhaft erweisen, wie u.a. mit äthylenbilden Stoffen verschiedener chemischer Struktur (z.B. Phosphonsäurederivate und Silane, Äthylhydrazine), Oniumverbindungen (z.B. Trimethylammonium-, Hydrazonium- und Sulfoniumsalze, Derivate von Morpholinium-, Piperidinium- und Pyridaziniumverbindungen). Von Interesse sind auch weitere wachstumsregulierende Substanzen u.a. aus der Gruppe der Trifluormethylsulfonamido-p-acetotoluidide, Maleinsäurehydrazid, Abscissinsäurederivate, chlorierte Phenoxyfettsäuren mit auxinähnlicher Wirkung, sowie höherwertige Alkohole und Fettsäureester mit spezifischer Wirkung auf meristematische Gewebepartien.

Die erfindungsgemäßen Mittel können in ihrer aufgewandten Menge schwanken. Die aufgewandte Menge hängt hauptsächlich von der Art des gewünschten Effektes ab.

Die Aufwandmenge liegt im allgemeinen zwischen 0,1 und 15 oder mehr, vorzugsweise 0,2 und 6 kg Wirkstoff pro Hektar.

Sie beeinflussen das Wachstum ober- und unterirdischer Pflanzenteile in verschiedener Weise und besitzen in den üblichen Anwendungskonzentrationen eine geringe Warmblütertoxizität.

Die neuen Mittel greifen in die physiologischen Vorgänge der Pflanzenentwicklung ein und können für verschiedene Zwecke verwendet werden. Die verschiedenartigen Wirkungen dieser Wirkstoffe hängen im wesentlichen ab von dem Zeitpunkt der Anwendung, bezogen auf das Entwicklungsstadium des Samens oder der Pflanze, sowie von den angewendeten Konzentrationen.

Mit den neuen Mitteln wird das vegetative und generative Pflanzenwachstum, sowie bei entsprechender Konzentration auch die Keimfähigkeit beeinflußt.

Die Beeinflussung der vegetativen Entwicklung besteht insbesondere in einer Reduzierung der Wuchshöhe, wodurch bei zahlreichen Pflanzen, insbesondere Getreide, eine erhöhte Standfestigkeit und verringerte Tendenz zu sogenanntem "Lagern" bewirkt wird. Gleichzeitig wird die Bestockung verbessert, was zu einer höheren Zahl ährentragender Halme je Flächeneinheit führt.

Bei Gras wirkt sich die reduzierte Wuchshöhe in einer dichteren, resistenteren Narbe und vor allem in der Einsparung mehrerer Schnitte aus. Letzteres ist für Zierrasen, aber auch für Grasflächen entlang der Straßen und in Parkanlagen von großem arbeitswirtschaftlichen Vorteil. Hinzu kommt, daß parallel zur Wuchshöhenreduzierung eine Zunahme des Chlorophyllgehaltes stattfindet, wodurch behandelte Grasflächen, aber auch sonstige Pflanzenbestände, eine deutlich dunklere grüne Färbung annehmen.

Die Beeinflussung des vegetativen Wachstums bewirkt bei zahlreichen Pflanzen, wie z.B. Baumwolle und Soja, eine starke Zunahme des Blüten- und Fruchtansatzes.

Besonders hervorzuheben ist die überraschende Erscheinung, daß das Wurzelwachstum durch die Behandlung mit den erfindungsgemäßen Substanzen gefördert wird. Dies führt zu einer rationelleren Wasser- und Nährstoffausnutzung durch die behandelten Pflanzen; dabei wird nicht nur die Trockenheitssonder auch die Kälteresistenz (Frostresistenz) erhöht.

Vielseitig und umfangreich sind auch die Anwendungsmöglichkeiten im Obst- und Zierpflanzenbau, sowie im Landschaftsbau einschließlich der sachgemäßen Vegetationsbeeinflussung auf Brachflächen, sowie auf Flug- und Übungsplätzen.

Bei der Beeinflussung von Blüh- und Reifevorgangen, sowie im Rahmen spezieller Anzuchtverfahren, lassen sich die Substanzen ebenfalls erfolgreich anwenden.

Nicht zuletzt können die neuen Mittel auch die Konzentration wichtiger Pflanzeninhaltsstoffe wie Zucker und Proteine positiv beeinflussen.

Das Ausmaß und die Art der Wirkung sind von verschiedenen Faktoren abhängig, insbesondere von Applikationszeit in bezug auf das Entwicklungsstadium der Pflanze und der Anwendungskonzentration. Diese Faktoren sind aber wiederum je nach Pflanzenart und dem gewünschten Effekt verschieden. So wird man beispielsweise Rasenflächen während der gesamten Wachstumsperiode behandeln; Zierpflanzen, bei denen die Intensität und Anzahl der Blüten erhöht werden soll, vor Ausbildung der Blütenenlage; Pflanzen, deren Früchte verwendet resp. verwertet werden, in entsprechendem

Abstand vor der Ernte. Verschiedene Derivate der hier beschriebenen Verbindungsklasse haben herbizide Eigenschaften. Sie eignen sich daher zur Beseitigung und Niederhaltung von unerwünschtem Pflanzenwuchs.

Beispiel 9

Einfluß von einigen polycyclischen, stickstoffhaltigen Verbindungen auf das Längenwachstum von Weizen und Gerste.

Die beiden Sommergetreidearten -Weizen ("Opal") und Gerste (Sorte "Union")—wurden unter Gewächshausbedingungen in Kunststoffschalen von 11,5 cm Durchmesser in Torfkultursubstrat, das ausreichend mit Nährstoffen versorgt war, im Winterhalbjahr angezogen.

Zur Bodenbehandlung (Vorauflaufbehandlung) wurden die Wirkstoffe als wäßrige Aufbereitungen in zwei Aufwandmengen nach der Einsaat auf die Endoberfläche gegossen.

Die Blattbehandlung erfolgte durch Besprühen der Pflanzen bei einer Wuchshöhe von 11 cm mit wäßrigen Aufbereitungen der Wirkstoffe in zwei Aufwandmengen.

Die durch die Behandlung hervorgerufenen Kürzungen der Wuchshöhe wurden durch Längenmessung der Pflanzen bei Versuchsende nach 30 Tagen Wachstumszeit ermittelt und zur Wuchshöhe der unbehandelten Pflanzen in Relation gesetzt.

Dabei konnte die einkürzende Wirkung der geprüften Substanzen gezeigt werden. Zum Vergleich wurde der bekannte Wirkstoff N,N,N-Trimethyl-N-$\beta$-chloräthylammoniumchlorid (CCC, DE—B—12 94 734) verwendet.

Einzelergebnisse sind den nachfolgenden Tabellen zu entnehmen.

Beeinflussung des Längenwachstums von Sommerweizen Bodenbehandlung

| Wirkstoff | Aufwandmenge kg/ha | Pflanzenlänge cm | % |
|---|---|---|---|
| Kontrolle (unbehandelt) | – | 30,0 | 100 |
| CCC | 3 | 21,5 | 71,7 |
| (bekannt) | 12 | 19,5 | 65,0 |
| 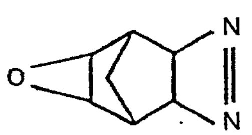 | 3 | 21,5 | 71,7 |
| | 12 | 12,5 | 41,7 |
| 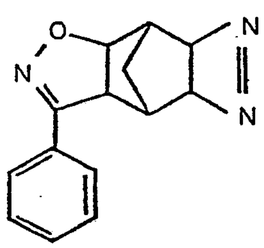 | 3 | 20,0 | 66,7 |
| | 12 | 14,0 | 46,7 |

Beeinflussung des Längenwachstums von Sommergerste A Bodenbehandlung

| Wirkstoff | Aufwandmenge kg/ha | Pflanzenlänge cm | % |
|---|---|---|---|
| Kontrolle (unbehandelt) | – | 32,2 | 100 |
| CCC bekannt | 3 | 27,5 | 85,1 |
| | 12 | 24,5 | 75,9 |
| 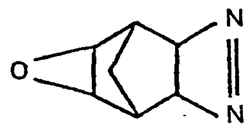 | 3 | 25,0 | 77,4 |
| | 12 | 14,0 | 43,3 |
| 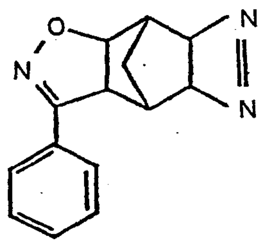 | 3 | 24,5 | 75,9 |
| | 12 | 16,0 | 49,5 |

Beispiel 10

Einfluß von einigen polycyclischen, stickstoffhaltigen Verbindungen auf das Längenwachstum von Weizen und Gerste.

Sommerweizen (Sorte "Opal") und Sommergerste (Sorte "Union") wurden in Kunststoffschalen von 11,5 cm Durchmesser in ausreichend mit Nährstoffen versorgtem Torfkultursubstrat unter Gewächshausbedingungen angezogen.

Die Wirkstoffe wurden zur Bodenbehandlung (Vorauflaufbehandlung) als wäßrige Aufbereitungen in zwei Aufwandmengen nach der Einsaat auf die Substratoberfläche gegossen.

Zur Blattbehandlung wurden die Testpflanzen bei einer Wuchshöhe von 10 cm mit wäßrigen Aufbereitungen der Wirkstoffe in zwei Aufwandmegen besprüht.

Bei Versuchsende wurden die Pflanzenlängen gemessen. Die ermittelten Daten zeigen die einkürzende Wirkung der geprüften Wirkstoffe. Zum Vergleich wurde der bekannte Wirkstoff ß-Chloräthylphosphonsäure (Ethephon, DE—B—16 67 968) verwendet.

Einzelergebnisse sind den nachfolgenden Tabellen zu entnehmen.

Beeinflussung des Langenwachstums von Sommerweizen A Bodenbehandlung

| Wirkstoff | Aufwandmenge kg/ha | Pflanzenlänge cm | % |
|---|---|---|---|
| Kontrolle (unbehandelt) | – | 32,0 | 100 |
| Ethephon | 3 | 29,0 | 90,6 |
| bekannt | 12 | 25,0 | 78,1 |
| 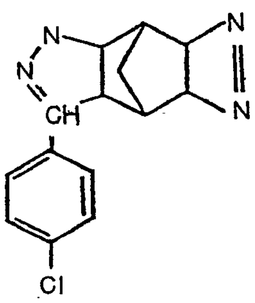 | 3 | 17,0 | 53,1 |
| | 12 | 14,0 | 43,8 |
| 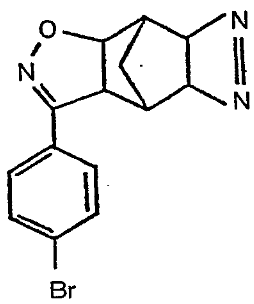 | 3 | 26,5 | 82,8 |
| | 12 | 25,5 | 79,7 |
| 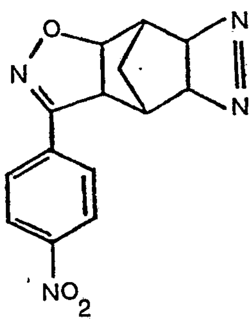 | 3 | 25,5 | 79,7 |
| | 12 | 22,0 | 68,8 |
| 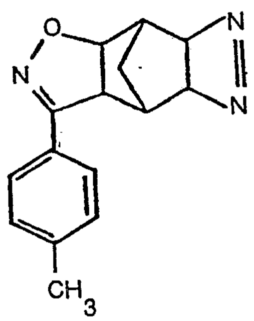 | 3 | 23,5 | 73,4 |
| | 12 | 22,0 | 68,8 |

| Wirkstoff | Aufwandmenge kg /ha | Pflanzenlänge cm | % |
|---|---|---|---|
| 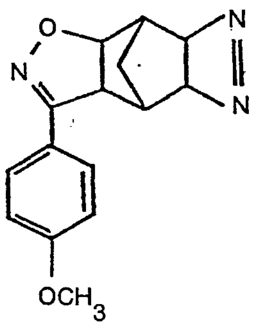 | 3 | 27,0 | 84,4 |
| | 12 | 24,5 | 76,6 |
| 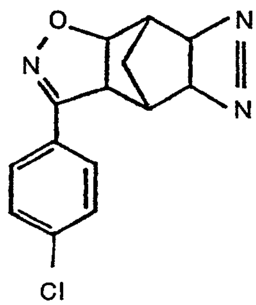 | 3 | 16,0 | 50,0 |
| | 12 | 15,0 | 46,9 |

Beeinflussung des Längenwachstums von Sommerweizen B Blattbehandlung

| Wirkstoff | Aufwandmenge kg /ha | Pflanzenlänge cm | % |
|---|---|---|---|
| Kontrolle (unbehandelt) | – | 32,8 | 100 |
| Ethephon | 1,5 | 30,0 | 91,5 |
| bekannt | 6,0 | 25,0 | 76,2 |
| 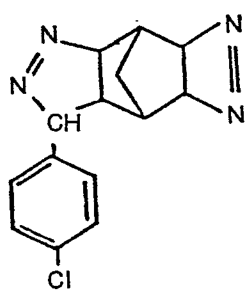 | 1,5 | 23,5 | 71,6 |
| | 6,0 | 22,0 | 67,1 |
| 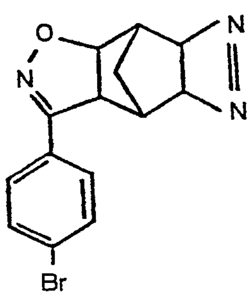 | 1,5 | 29,0 | 88,4 |
| | 6,0 | 27,5 | 83,8 |

| Wirkstoff | Aufwandmenge kg/ha | Pflanzenlänge cm | % |
|---|---|---|---|
| 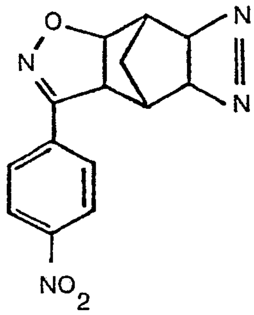 | 1,5<br>6,0 | 29,0<br>27,0 | 88,4<br>82,3 |
| 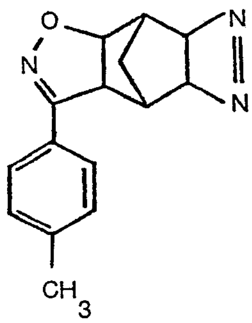 | 1,5<br>6,0 | 29,5<br>27,5 | 89,9<br>83,3 |
| 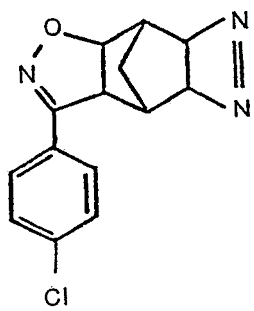 | 1,5<br>6,0 | 29,0<br>27,0 | 88,4<br>82,3 |
| 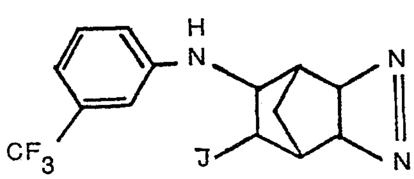 | 1,5<br>6,0 | 27,5<br>25,5 | 83,3<br>77,7 |

Beeinflussung des Längenwachstums von Sommergerste A Bodenbehandlung

| Wirkstoff | Aufwandmenge kg/ha | Pflanzenlänge cm | % |
|---|---|---|---|
| Kontrolle (unbehandelt) | – | 34,3 | 100 |
| Ethephon | 3 | 32,0 | 93,3 |
| bekannt | 12 | 27,5 | 80,2 |
| 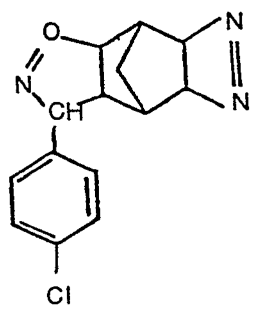 | 3 | 22,0 | 64,1 |
| | 12 | 20,0 | 58,3 |
| 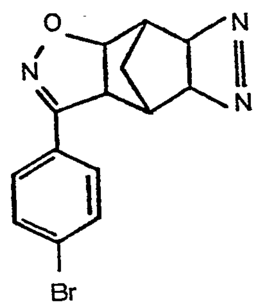 | 3 | 28,0 | 81,6 |
| | 12 | 26,0 | 75,8 |
| 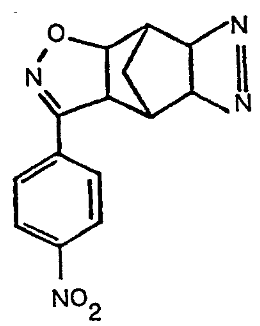 | 3 | 27,0 | 78,7 |
| | 12 | 24,0 | 70,0 |
| 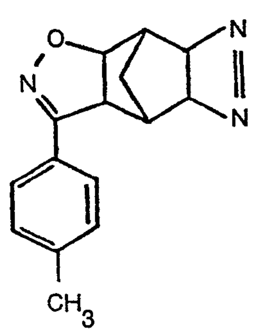 | 3 | 25,0 | 72,9 |
| | 12 | 21,0 | 61,2 |

| Wirkstoff | Aufwandmenge kg /ha | Pflanzenlänge cm | % |
|---|---|---|---|
| 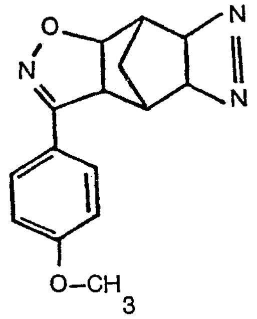 | 3 | 30,0 | 87,5 |
| | 12 | 29,0 | 84,5 |
| 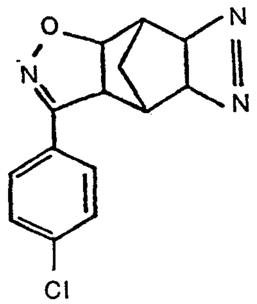 | 3 | 26,0 | 75,8 |
| | 12 | 24,0 | 70,0 |

Beeinflussung des Längenwachstums von Sommergerste B Blattbehandlung

| Wirkstoff | Aufwandmenge kg /ha | Pflanzenlänge cm | % |
|---|---|---|---|
| Kontrolle (unbehandelt) | – | 28,0 | 100 |
| Ethephon | 1,5 | 27,0 | 96,4 |
| bekannt | 6,0 | 24,5 | 87,5 |
| 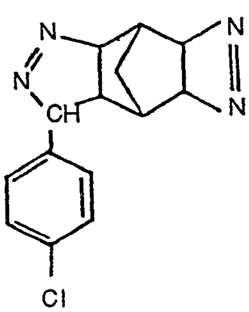 | 1,5 | 22,0 | 78,6 |
| | 6,0 | 19,5 | 69,6 |

| Wirkstoff | Aufwandmenge kg /ha | Pflanzenlänge cm | % |
|---|---|---|---|
| 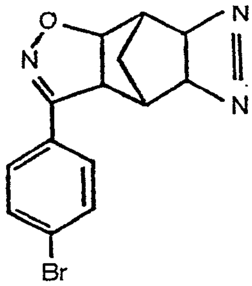 | 1,5 | 25,5 | 91,1 |
| | 6,0 | 25,0 | 89,3 |
| 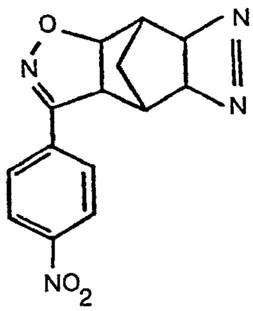 | 1,5 | 25,0 | 89,3 |
| | 6,0 | 24,5 | 87,5 |
| 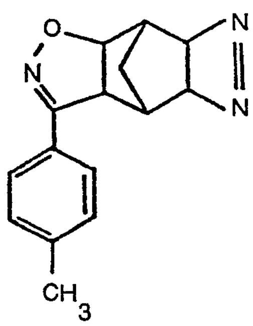 | 1,5 | 25,5 | 91,1 |
| | 6,0 | 25,0 | 89,3 |
| 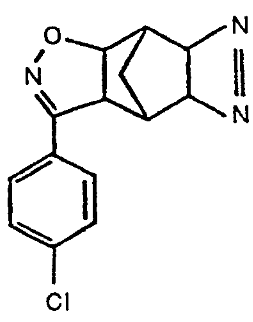 | 1,5 | 26,0 | 92,9 |
| | 6,0 | 24,5 | 87,5 |
| 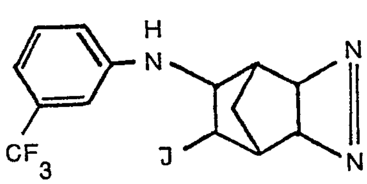 | 1,5 | 25,0 | 89,3 |
| | 6,0 | 24,0 | 85,7 |

Beispiel 11

Einfluß von einigen polycyclischen, stickstoffhaltigen Verbindungen auf das Längenwachstum von Weizen, Gerste und Roggen.

Die Testpflanzen—Sommerweizen (Sorte "Opal"), Sommergerste ("Union") und Sommerroggen ("Petkuser")—wurden unter Gewächshausbedingungen in Kunststoffschalen von 11,5 cm Durchmesser angezogen. Als Kultursubstrat diente Torf, der ausreichend mit Nährstoffen versorgt war.

Nach der Einsaat erfolgte die Bodenbehandlung (Vorauflaufbehandlung). Dazu wurden die Wirkstoffe als wäßrige Aufbereitungen in vier Aufwandmengen auf die Substratoberfläche gegossen.

Die Blattbehandlung wurde durch Besprühen der Pflanzen bei einer Wuchshöhe von 11 cm mit wäßrigen Aufbereitungen der Wirkstoffe in drei Aufwandmengen durchgeführt.

Bei Versuchsende wurde die beobachtete einkürzende Wirkung der Prüfsubstanzen durch Längenmessung der Pflanzen belegt.

Zum Vergleich wurde der bekannte Wirkstoff N,N,N-Trimethyl-N-ß-chloräthylammoniumchlorid (CCC, DE—B—12 94 734) verwendet.

Einzelergebnisse sind den nachfolgenden Tabellen zu entnehmen.

Beeinflussung des Längenwachstums von Sommerweizen Blattbehandlung

| Wirkstoff | Aufwandmenge kg/ha | Pflanzenlänge cm | % |
|---|---|---|---|
| Kontrolle (unbehandelt) | — | 30,8 | 100 |
| CCC bekannt | 0,75 | 27,5 | 89,3 |
| | 1,50 | 26,0 | 84,4 |
| | 3,00 | 25,0 | 81,2 |
| 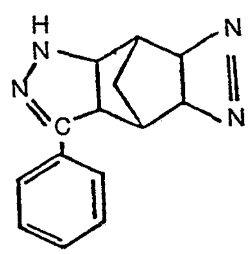 | 0,75 | 26,5 | 86,0 |
| | 1,50 | 25,5 | 82,8 |
| | 3,00 | 24,0 | 77,9 |

Beeinflussung des Längenwachstums von Sommergerste

A Bodenbehandlung

| Wirkstoff | Aufwandmenge kg /ha | Pflanzenlänge cm | % |
|---|---|---|---|
| Kontrolle | – | 32,8 | 100 |
| (unbehandelt) | | | |
| CCC | 0,75 | 29,5 | 89,9 |
| bekannt | 1,50 | 29,5 | 89,9 |
| 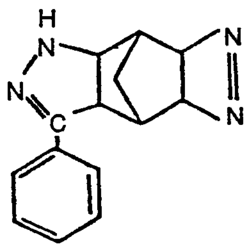 | 3,00 | 27,5 | 83,8 |
| | 6,00 | 26,0 | 79,3 |
| | 0,75 | 32,0 | 97,6 |
| | 1,50 | 28,0 | 85,4 |
| | 3,00 | 27,0 | 82,3 |
| | 6,00 | 23,0 | 70,1 |

B Blattbehandlung

| Wirkstoff | Aufwandmenge kg /ha | Pflanzenlänge cm | % |
|---|---|---|---|
| Kontrolle | – | 27,8 | 100 |
| (unbehandelt) | | | |
| CCC | 0,75 | 27,5 | 98,4 |
| bekannt | 1,50 | 27,0 | 97,1 |
| | 3,00 | 27,0 | 97,1 |
| 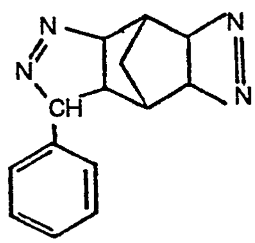 | 0,75 | 23,5 | 84,5 |
| | 1,50 | 23,0 | 82,7 |
| | 3,00 | 21,5 | 77,3 |
| 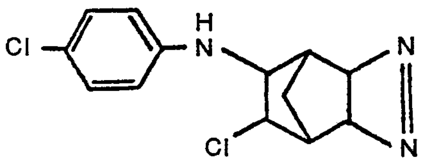 | 0,75 | 26,0 | 93,5 |
| | 1,50 | 25,5 | 91,7 |
| | 3,00 | 23,5 | 84,5 |

Beeinflussung des Längenwachstums von Sommerroggen

A Bodenbehandlung

| Wirkstoff | Aufwandmenge kg /ha | Pflanzenlänge cm | % |
|---|---|---|---|
| Kontrolle | – | 32,3 | 100 |
| (unbehandelt) | | | |
| CCC | 0,75 | 31,5 | 97,5 |
| bekannt | 1,50 | 30,5 | 94,4 |
| | 3,00 | 30,0 | 92,9 |
| | 6,00 | 28,5 | 88,2 |
| 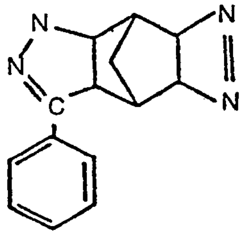 | 0,75 | 30,0 | 92,9 |
| | 1,50 | 26,5 | 82,0 |
| | 3,00 | 25,0 | 77,4 |
| | 6,00 | 23,0 | 71,2 |
| 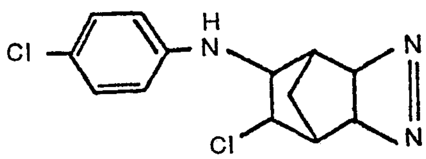 | 0,75 | 28,0 | 86,7 |
| | 1,50 | 27,0 | 83,5 |
| | 3,00 | 25,0 | 77,4 |
| | 6,00 | 23,0 | 71,2 |

B Blattbehandlung

| Wirkstoff | Aufwandmenge kg /ha | Pflanzenlänge cm | % |
|---|---|---|---|
| Kontrolle | – | 28,5 | 100 |
| (unbehandelt) | | | |
| CCC | 0,75 | 27,0 | 94,7 |
| bekannt | 1,50 | 25,5 | 89,5 |
| | 3,00 | 25,5 | 89,5 |
| 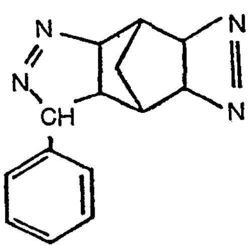 | 0,75 | 23,0 | 80,7 |
| | 1,50 | 20,5 | 71,9 |
| | 3,00 | 20,5 | 71,9 |
| 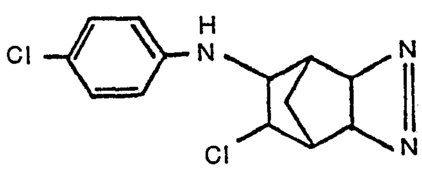 | 0,75 | 25,5 | 89,5 |
| | 1,50 | 24,5 | 86,0 |
| | 3,00 | 24,5 | 86,0 |

Beispiel 12

Wirkung zu Gras bzw. Rasen.

In einem Versuch in Großgefäßen wurde auf einem Lehmboden Rasen der folgenden Standard-Mischung angesät: Agrostis tenuis 10%, Cynosurus cristatus 10%, Festuca rubra 15%, Lolium perenne 35% und Poa pratensis 30%. Die Düngung wurde mit 1,5 g N als Ammonnitrat und 1 g $P_2O_5$ als sek. Kaliumphosphat vorgenommen. Nach 2 vorangegangenen Schnitten wurden die Wirkstoffe in verschiedenen Aufwandmengen bei einer Wuchshöhe von 4 cm in üblicher Weise gespritzt. 19 Tage nach der Behandlung wurden Wuchshöhe und Grünmasse-ertrag bestimmt. Gegenüber der Kontrolle reagierten die Pflanzen aus der Behandlungsreihe mit sehr starken Kürzungen und einer entsprechend verminderten Grünmasseproduktion.

Aus der folgenden Tabelle ist auch die gute Dauerwirkung der geprüften Mittel zu sehen.

Beeinflussung des Längenwachstums und des Grünmasseertrages bei Gras

| Wirkstoff | Aufwandmenge kg/ha | 1. Schnitt Wuchshöhe cm | relativ | Frischgewicht g | relativ | 2. Schnitt Wuchshöhe cm | relativ | Frischgewicht g | relativ |
|---|---|---|---|---|---|---|---|---|---|
| Kontrolle | – | 21,8 | 100 | 134,2 | 100 | 20,0 | 100 | 90,6 | 100 |
| 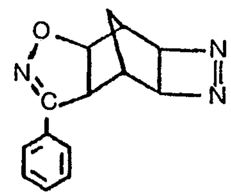 | 3,0 | 20,0 | 91,7 | 116,3 | 86,7 | 19,0 | 95,0 | 94,8 | 104,6 |
| | 6,0 | 17,0 | 78,0 | 101,1 | 75,3 | 16,5 | 82,5 | 85,2 | 94,0 |
| 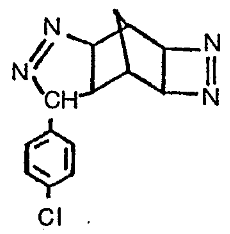 | 3,0 | 20,0 | 91,7 | 125,1 | 93,2 | 19,0 | 95,0 | 89,9 | 99,2 |
| | 6,0 | 18,0 | 82,6 | 119,0 | 88,7 | 19,0 | 95,0 | 92,2 | 101,8 |
| 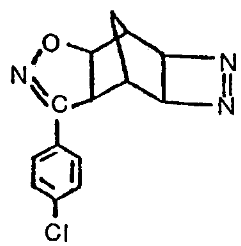 | 3,0 | 21,0 | 96,3 | 117,7 | 87,7 | 18,5 | 92,5 | 75,3 | 83,1 |
| | 6,0 | 18,5 | 84,9 | 114,0 | 84,9 | 17,0 | 85,0 | 72,1 | 79,6 |

Beispiel 13

Versuche zur Bestimmung der wachstumsregulierenden oder herbiziden Wirkung.

Zur Demonstration der Wirksamkeit neuer Verbindungen dienten Gewächshausversuche, welche nach folgender Methode durchgeführt wurden: Plastikblumentöpfe von 300 $cm^3$ Inhalt wurden mit lehmigem Sand gefüllt und mit den Testpflanzen nach Arten getrennt bestückt. Es handelt sich um die Einsaat von Samen, ein Verpflanzen von vegetativ vermehrten Arten oder um die Bereitstellung von getopften Pflanzen (z.B. Reben). Bei Vorauflaufanwendung wurden die Wirkstoffe unmittelbar nach der Einsaat in Wasser als Verteilungsmittel suspendiert oder emulgiert mittels feinverteilender Düsen auf die Erdoberfläche ausgebracht. Die Topfreben wurden durch Angießen mit der entsprechend verdünnten Brühe behandelt. Bei Nachauflaufbehandlung wurden die Mittel ebenfalls, wie oben ausgeführt, in Wasser auf die Blätter der Testpflanzen gespritzt, wobei man auch noch die freie Bodenoberfläche traf. In einigen Spezialfällen wurden in fester Form vorliegende Wirkstoffe erst in DMF (Dimethylformamid) aufgelöst und in einer Gesamtbrühmenge von 400 l/ha Wasser ausgebracht. Für die Nachauflaufbehandlung zog man die Pflanzen je nach Wuchsform in den Versuchsgefäßen bis zu einer Höhe von 3 bis 10 cm heran und behandelte sie danach. Bei Gräsern wurden zuweilen der nach der Einsaat zustandegekommene Erstaustrieb abgeschnitten und erst der Neuaustrieb 2—3 Tage nach dem Schneiden behandelt. Den Temperaturansprüchen der Testpflanzen kam man insofern entgegen, als man sie in kühleren oder wärmeren Sektionen der Gewächshausanlagen aufstellte. Die Versuchsperiode dauerte 4 bis 6 Wochen. Während dieser Zeit wurden die Pflanzen gepflegt und ihre Reaktion auf die einzelnen Behandlungen ausgewertet.

Die Aufwandmengen der Prüfsubstanzen verstehen sich in kg/ha Aktivsubstanz unabhängig vom Anwendungsverfahren oder der eingesetzten Wassermenge.

Für die Auswertung diente eine Skala von 0 bis 100. Dabei bedeutete 0 = normaler Aufgang oder keine Schädigung bzw. keine Hemmung, 100 = kein Auflaufen der Pflanzen oder völliges Absterben. Die Wuchsbeeinflussung ist dabei zu differenzieren zwischen erwünschten Veränderungen ohne nennenswerte nachteilige phytotoxische Erscheinungen (z.B. rein wachstumsregulierende Effekte) und solchen mit phytotoxischem herbizidem Charakter.

Die Ergebnisse gehen aus den Tabellen hervor. Sie präsentieren Verbindungen mit ausgeprägten wachstumsregulierenden Eigenschaften und Substanzen, welche als Herbizide Verwendung finden können.

Liste der Pflanzenarten

| Lateinischer Name | Deutscher Name | Englischer Name |
|---|---|---|
| Alopecurus myosuroides | Ackerfuchsschwanz | Blackgrass |
| Cynodon dactylon | Hundszahngras | Bermudagrass |
| Cyperus esculentus | Erdmandel | Yellos nutsedge |
| Datura stramonium | Stechapfel (gemeiner) | Jimson weed |
| Digitaria sanguinalis | Blutfingerhirse | Hairy crabgrass |
| Echinochloa crus galli | Hühnerhirse | Barnyardgrass |
| Euphorbia spp. meist E geniculata | Wolfsmilcharten | Spurge family |
| Eleusine indica | — | Goosegrass |
| Galium aparine | Klettenlabkraut | Catchweed bedstraw |
| Ipomoea spp. meist I. lacunosa | Prunkwinde | Morningglory |
| Matricaria chamomilla | Echte Kamille | Wild chamomile |
| Panicum virgatum | Rutenhirse | Switchgrass |
| Poa annua | Einjährige Rispe | Annual bluegrass |
| Setaria faberii | Borstenhirse | Giant foxtail |
| Sinapis alba | Weißer Senf | White mustard |
| Solanum nigrum | Schwarzer Nachtschatten | Black nightshade |
| Sorghum halepense | Sudangras | Johnsongrass |
| Stellaria media | Vogelsternmiere | Chickweed |

TABELLE – Verträglichkeit neuer Verbindungen für eingetopfte Kulturreben im Gewächshaus bei Anwendung im Gießverfahren

| Verbindungen | ka /ha | Reaktion des Triebzuwachses cm | Topfreben Blattschäden |
|---|---|---|---|
| 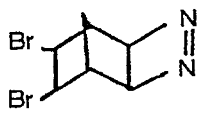 | 4,0 | 14,7 | keine |
| | 8,0 | 13,3 | |
| 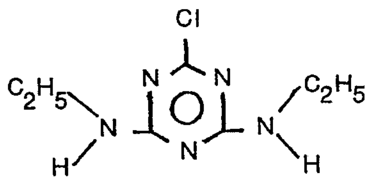 | 4,0 | 17,3 | chlorotische Aufhellungen, |
| | 8,0 | 10,3 | Nekrosen |
| bekannt | | | |
| 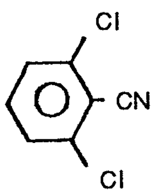 | 4,0 | 15,3 | leichte Chlorose |
| | 8,0 | 15,7 | |
| bekannt | | | |
| unbehandelte Kontrolle | – | 15,3 | keine |

TABELLE – Beseitigung und Hemmung unerwünschter Pflanzen bei Vor- und Nachauflaufanwendung (VA, NA) im Gewächshaus

| Verbindung | kg /ha | Testpflanzen und % Schädigung<br>Cyper. esc. | Echin. c.g. | Lam. spp. | Setar. spp. | Solan. nig. |
|---|---|---|---|---|---|---|
| 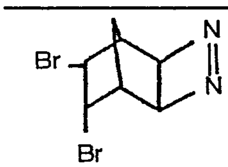 | 2,0 VA | 20 | 50 | 95 | 100 | 95 |
| | 4,0 VA | 55 | 80 | 95 | 100 | 95 |
| | 8,0 VA | 70 | 90 | 95 | 100 | 95 |
| 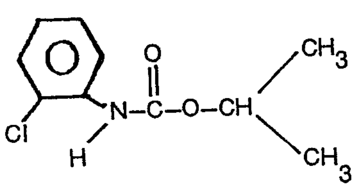 | 2,0 VA | 0 | 88 | 72 | 40 | 85 |
| | 4,0 VA | 0 | 90 | 90 | 100 | 85 |
| | 8,0 VA | 0 | 95 | 95 | 100 | 90 |
| bekannt | | | | | | |

0 = keine Schädigung

100 = Pflanzen abgestorben

TABELLE – Hemmung von Rasengras durch neue Verbindungen bei Vorauflaufanwendung im Gewächshaus

| Verbindung | kg/ha | Wuchshemmung % nach dem Auflaufen Poa pratensis | |
|---|---|---|---|
| 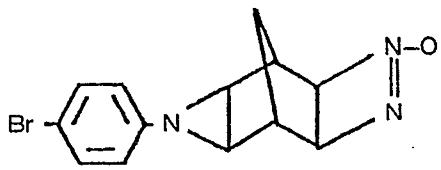 | 0,5 | 50 | durchgehend sehr |
| | 1,0 | 60 | kurz gewachsen, |
| | 2,0 | 60 | kompakte, dunkelgrüne |
| | 4,0 | 70 | Pflanzen |
| bekannt | 0,5 | 0 | |
| | 1,0 | 10 | |
| | 2,0 | 10 | |
| $[Cl-CH_2-CH_2-\overset{\oplus}{N}(CH_3)_3]\overset{\ominus}{Cl}$ | 4,0 | 10 | |

0 = keine Hemmung

100 = kein Wuchs

TABELLE – Beeinflussung des Sproßwachstums verschiedener Pflanzen bei Nachauflaufanwendung im Gewächshaus

| Verbindung | kg/ha | Sproßlänge cm bei Arachys hypogaea | Oryza sativa | Poa pratensis |
|---|---|---|---|---|
| 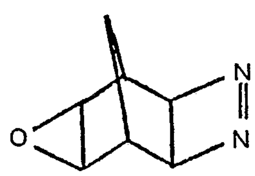 | 1,0 | 10 | 16 | 2 x |
| | 2,0 | 10 | 16 | 2 x |
| | 4,0 | 10 | 16 | 2 x |
| Kontrolle (unbehandelt) | | 14 | 22 | 4 |
| 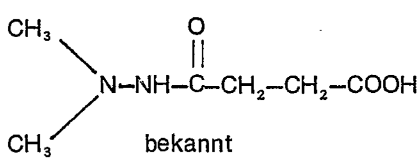 | 1,0 | 12 | – | 4 |
| | 2,0 | 5 | – | 4 |
| bekannt | 4,0 | 5 | – | 4 |
| bekannt | 1,0 | 16 | 20 | 4 |
| | 2,0 | 16 | 20 | 4 |
| $[Cl-CH_2-CH_2-\overset{\oplus}{N}(CH_3)_3]\overset{\ominus}{Cl}$ | 4,0 | 16 | 20 | 3 |

x gewisse Blattverbrennungen

TABELLE – Beeinflussung des Sproßwachstums verschiedener Pflanzen bei Nachauflaufbehandlung im Gewächshaus

| Strukturformel | kg/ha | Sproßlänge cm bei Cynodon[x] dactylon | Euphorbia geniculata | Hordeum vulgare | Poa pratensis |
|---|---|---|---|---|---|
| 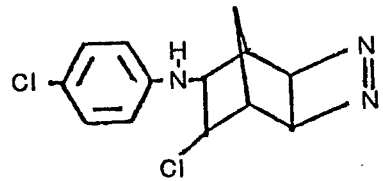 | 0,5 | – | 9 | 12,5 | – |
| | 1,0 | 5 | 9 | 12 | – |
| | 2,0 | 5 | 9 | 12 | – |
| 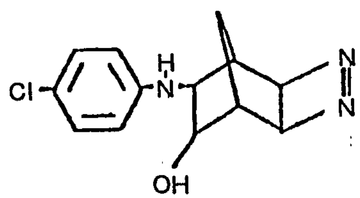 | 0,5 | – | – | 16 | – |
| | 1,0 | 6 | – | 15 | 13 |
| | 2,0 | 6 | – | 14 | 13 |
| $[Cl-CH_2-CH_2-\overset{\oplus}{N}(CH_3)_3]\overset{\ominus}{Cl}$ bekannt | 0,5 | – | 12,5 | 14 | – |
| | 1,0 | 7 | 13 | 14 | 20 |
| | 2,0 | 7 | 13 | 14 | 19 |
| Unbehandelte Kontrolle | – | 8 | 14 | 19 | 20 |

[x] aus Samen

TABELLE – Hemmung des Sproßwachstums durch Wachstumsregulatoren bei Vor- und Nachauflaufanwendung (VA, NA) im Gewächshaus

| Strukturformel | kg /ha | Testpflanzen und Sproßhemmung % Euphorbia geniculata | Medicago sativa |
|---|---|---|---|
| 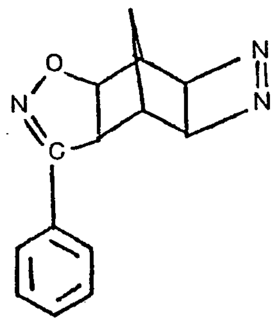 | 0,5 VA | 33 | – |
| | 1,0 VA | 40 | – |
| | 2,0 NA | 30 | 20 |
| | 4,0 NA | 30 | 20 |
| $[Cl-CH_2-CH_2-\overset{\oplus}{N}(CH_3)_3]\overset{\ominus}{Cl}$ | 0,5 VA | 0 | 0 |
| | 1,0 VA | 0 | 0 |
| bekannt | 2,0 NA | 0 | 0 |
| | 4,0 NA | 10 | 0 |

TABELLE – Beeinflussung des Pflanzenwachstums durch wachstumsregulierende Substanzen bei Vor- und Nachauflaufanwendung (VA, NA) im Gewächshaus

| Strukturformel | kg/ha | Arachys hypog. | Glycine max | Euphorbia genic. | Cynodon[x] dactyl. | Tritic. aestiv. | Hordeum vulg. | Poa prat. |
|---|---|---|---|---|---|---|---|---|
| | | Testpflanzen und % Sproßhemmung | | | | | | |
| 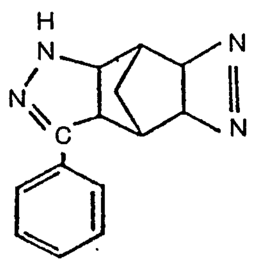 | 1,0 VA | 10 | 10 | 10 | 10 | 30 | 25 | 60 |
| | 2,0 VA | 10 | 25 | 30 | 10 | 30 | 25 | 60 |
| | 1,0 NA | 20 | – | – | 13 | 10 | 30 | 60 |
| | 2,0 NA | 30 | – | – | 25 | 20 | 30 | 60 |
| $[Cl-CH_2-CH_2-\overset{\oplus}{H}(CH_3)_3]\overset{\ominus}{Cl}$ | 1,0 VA | 0 | 0 | 0 | 0 | 20 | 20 | 0 |
| | 2,0 VA | 0 | 0 | 0 | 0 | 20 | 20 | 0 |
| bekannt | 1,0 NA | 0 | 0 | 0 | 0 | 30 | 10 | 0 |
| | 2,0 NA | 0 | 0 | 0 | 0 | 30 | 10 | 0 |
| 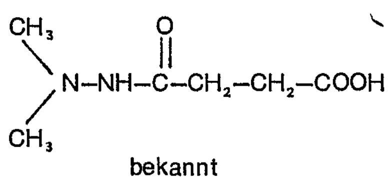 | 1,0 VA | 50 | 0 | 0 | – | – | – | – |
| | 2,0 VA | 50 | 0 | 0 | – | – | – | – |
| | 1,0 NA | 30 | – | – | 20 | – | – | – |
| bekannt | 2,0 NA | 30 | – | – | 20 | – | – | – |

[x] aus Samen

TABELLE – Herbizide Wirkung neuer Verbindungen bei Vor- oder Nachauflaufanwendung (VA, NA) im Gewächshaus

| Verbindungen | kg/ha | Testpflanzen und % Schädigung<br>Lolium mult. | Galium apar. | Ipom. spp. | Hentha piper. | Cynodon dact. (Samen) | Sinapis alba |
|---|---|---|---|---|---|---|---|
| 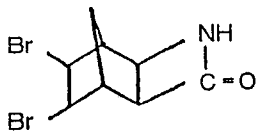 | 3,0 NA | 95 | 70 | 95 | 40 | – | – |
| 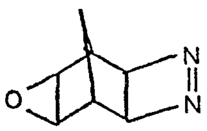 x | 3,0 VA | 95 | – | – | – | – | 90 |
| | 3,0 NA | 100 | 60 | 100 | 100 | – | – |
| 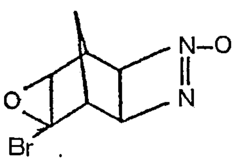 x | 3,0 NA | – | 30 | 100 | 40 | – | – |
| 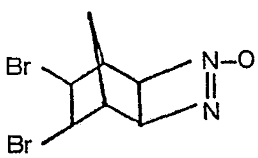 x | 3,0 VA | 80 | – | – | – | – | 80 |
| | 3,0 NA | 85 | 40 | 60 | 95 | – | – |
| 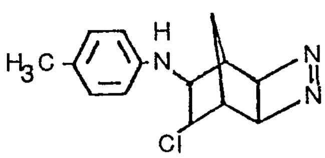 x | 3,0 VA | 80 | – | – | – | – | 100 |
| | 3,0 NA | 100 | 75 | 100 | 100 | – | – |
| 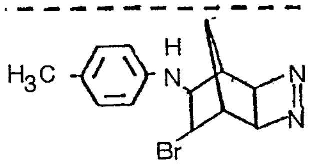 x | 3,0 VA | 95 | – | – | – | 2 kg/ha | 100 |
| | 3,0 NA | 100 | 80 | 100 | 100 | 100 | – |
| 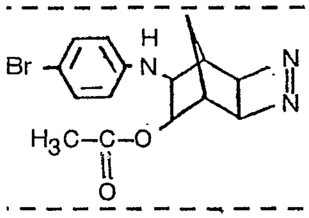 x | 3,0 VA | 80 | – | – | – | – | 100 |
| | 3,0 NA | 100 | 65 | 85 | 95 | – | – |
| 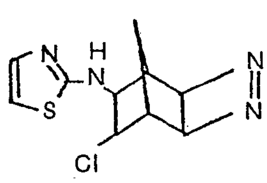 x | 3,0 NA | 100 | 80 | 80 | 100 | – | – |

x Auflösung in Dimethylformamid vor Ausbringung

TABELLE (Fortsetzung)

| Verbindungen | kg/ha | Testpflanzen und % Schädigung<br>Lolium mult. | Galium apar. | Ipom. spp. | Hentha piper. | Cynodon dact. (Samen) | Sinapis alba |
|---|---|---|---|---|---|---|---|
| 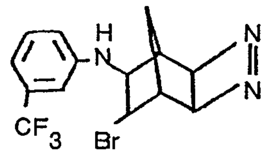 x | 3,0 VA | 100 | – | – | – | – | 95 |
| | 3,0 NA | 100 | 90 | 100 | 95 | – | – |
| 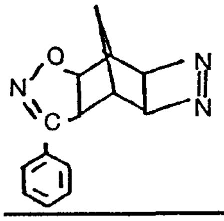 x | 3,0 VA | 100 | – | – | – | 2 kg/ha | 90 |
| | 3,0 NA | 100 | 95 | 100 | 75 | 100 | – |

x Auflösung in Dimethylformamid vor Ausbringung

Beispiel 14

20 Gewichtsteile des Wirkstoffs 1 werden in 3 Gewichtsteilen des Natriumsalzes der Diisobutylnaphthalin-α-sulfonsäure, 17 Gewichtsteilen des Natriumsalzes einer Ligninsulfonsäure aus einer Sulfitablauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 15

3 Gewichtsteile der Verbindung 2 werden mit 97 Gewichtsteilen feinteiligem Kaolin innig vermischt. Man erhält auf diese Weise ein Stäubemittel, das 3 Gewichtsprozent des Wirkstoffs enthält.

Beispiel 16

30 Gewichtsteile der Verbindung 2 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 17

40 Gewichtsteile des Wirkstoffs 1 werden mit 10 Teilen Natriumsalz eines Phenolsulfonsäureharnstoff-formaldehydkondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 18

20 Teile des Wirkstoffs 1 werden mit 2 Teilen Calciumsalz der Dodecylbenzolsulfonsäure, 8 Teilen Fettalkohol-polyglykoläther, 2 Teilen Natriumsalz eines Phenolsulfonsäureharnstoff-formaldehyd-kondensates und 68 Teilen eines paraffinischen Mineralöls innig vermischt. Man erhält eine stabile ölige Dispersion.

Beispiel 19

Man vermischt 90 Gewichtsteile der Verbindung 1 mit 10 Gewichtsteilen N-Methyl-α-pyrrolidon und erhält eine Lösung, die zur Anwendung in Form kleinster Tropfen geeignet ist.

Beispiel 20

20 Gewichtsteile der Verbindung 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und

feines Verteilen der Lösung in 100 000 Gewichtsteilen Wasser erhält man eine wäßrige Dispersion, die 0,02 Gewichtsprozent des Wirkstoffs enthält.

Beispiel 21

20 Gewichtsteile der Verbindung 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 22

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

**Patentansprüche**

1. Heterocyclische Norbornanderivate der Formel

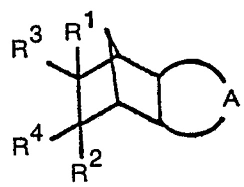

in der A die Reste 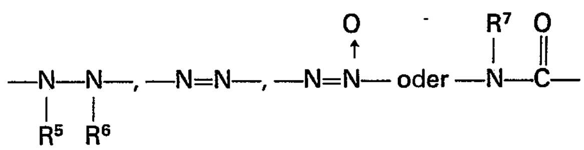 oder

bedeutet, wobei $R^5$ und $R^6$ je ein Wasserstoffatom oder einen Alkoxycarbonylrest und $R^7$ ein Wasserstoffatom oder die Gruppe —$SO_2Cl$ bedeutet und $R^1$ und $R^2$ entweder zusammen die Reste

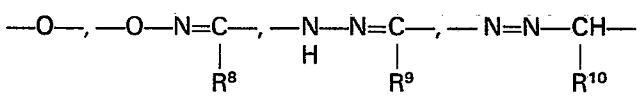 und, wenn A die Reste

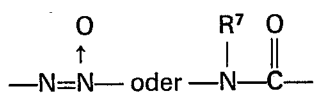 oder bedeutet, auch die Reste 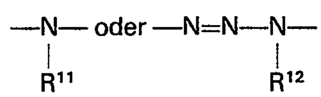 oder

bedeuten wobei die Reste

$R^8$ bis $R^{12}$ je einen Phenylrest, der durch Halogen, Nitro, Alkoxy, prim., sec. oder tert. Alkyl oder Halogenalkyl substituiert sein kann oder einen 2-Thiazolylrest bedeutet oder

$R^1$ und $R^2$ als einzelne Reste entweder je ein Halogenatom oder einer dieser Reste Halogen, OH oder Alkoxycarbonyl und der andere einen Rest der Formel, $R^{12}NH$— bedeutet und $R^3$ und $R^4$ je ein Wasserstoffatom bedeuten, wobei einer dieser Reste auch ein Halogenatom bedeuten kann, wenn $R^1$ und $R^2$ zusammen den Rest —O— bedeuten.

2. Verbindung der Formel

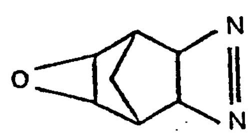

3. Verbindung der Formel

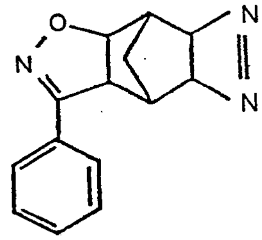

4. Verbindung der Formel

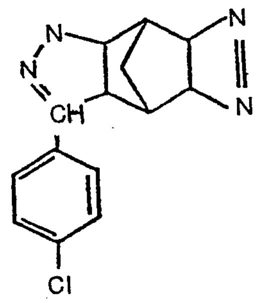

5. Verbindung der Formel

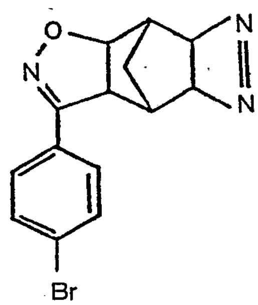

6. Verbindung der Formel

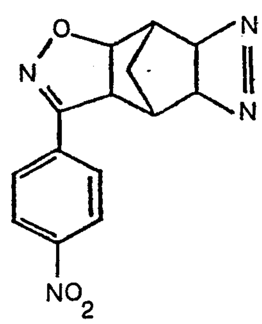

7. Verbindung der Formel

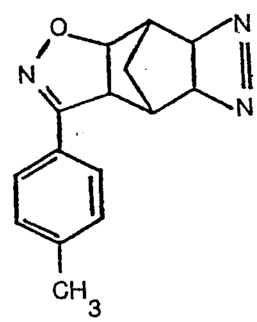

8. Verbindung der Formel

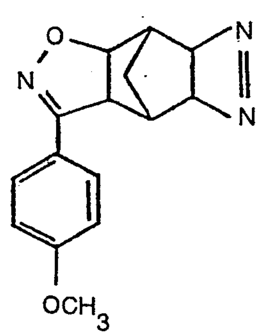

9. Verbindung der Formel

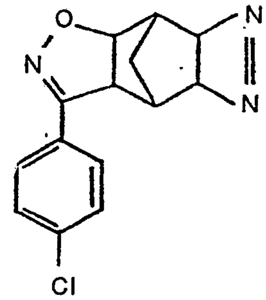

10. Verbindung der Formel

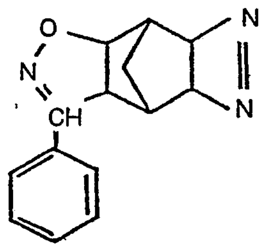

11. Verbindung der Formel

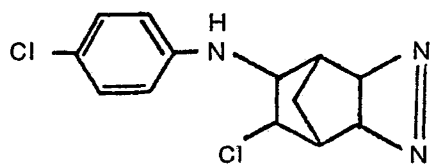

12. Mittel zur Beeinflussung des Pflanzenwachstums, enthaltend eine Verbindung gemäß Anspruch 1.

13. Mittel zur Beeinflussung des Pflanzenwachstums, enthaltend einen festen oder flüssigen Trägerstoff und eine Verbindung gemäß Anspruch 1.

14. Verfahren zur Herstellung eines Mittels zur Beeinflussung des Pflanzenwachstums, dadurch gekennzeichnet, daß man einen festen oder flüssigen Trägerstoff vermischt mit einer Verbindung gemäß Anspruch 1.

15. Verfahren zur Beeinflussung des Pflanzenwachstums, dadurch gekennzeichnet, daß man die Pflanze oder ihre Umgebung behandelt mit einer Verbindung gemäß Anspruch 1.

## Revendications

1. Dérivés hétérocycliques de norbornane de formule:

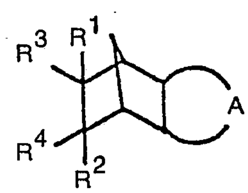

dans laquelle

A représente les restes $-N(R^5)-N(R^6)-$, $-N{=}N$, $-N(\rightarrow O){=}N$ ou $N(R^7)-C(=O)-$

$R^5$ et $R^6$ représentant chacun un atome d'hydrogène ou un reste alcoxycarbonyle et $R^7$ un atome d'hydrogène ou le groupe $-SO_2Cl$ et $R^1$ et $R^2$ ou bien représentent ensemble les restes

$-O-$, $-O-N{=}C(R^8)$, $-N(H)-N{=}C(R^9)-$, $-N{=}N-CH(R^{10})-$ et, lorsque A représente les restes

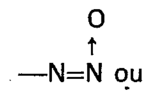 ou 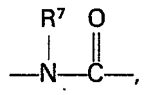, ils représentent aussi les restes 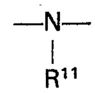 ou 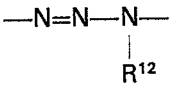

les restes $R^8$ à $R^{12}$ représentant chacun un reste phényle, qui peut être substitué par halogène, nitro, alcoxy, alkyle primaire, secondaire ou tertiaire, ou halogène-alkyle, ou bien représente un reste 2-thiazolyle ou

$R^1$ et $R^2$, comme restes séparés, représentent, soit, chacun un atome d'halogène, ou bien l'un de ces restes représente halogène OH ou alcoxycarbonyle et l'autre un reste de formule $R^{12}NH$— et

$R^3$, $R^4$ représentent chacun un atome d'hydrogène, un de ces restes pouvant également représenter un atome d'halogène lorsque $R^1$ et $R^2$ représentent ensembles le reste —O—.

2. Composé de formule

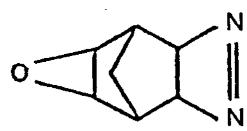

3. Composé de formule

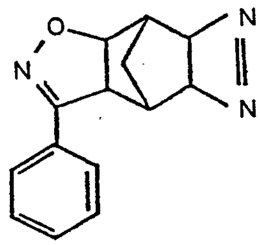

4. Composé de formule

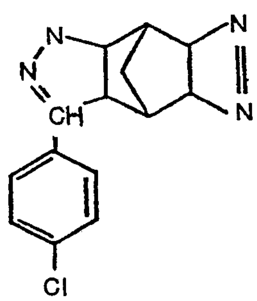

5. Composé de formule

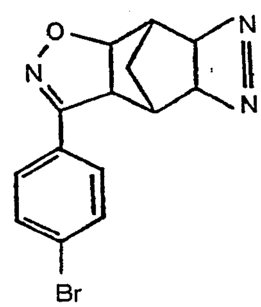

6. Composé de formule

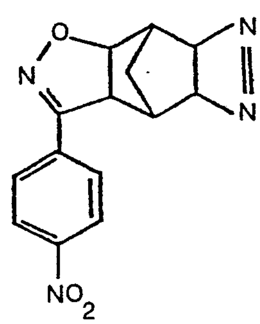

7. Composé de formule

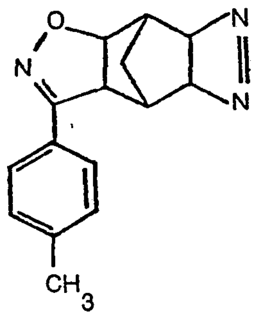

8. Composé de formule

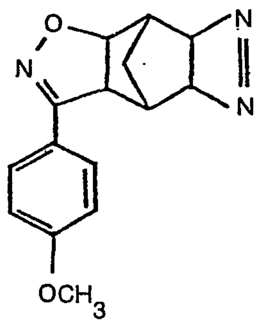

9. Composé de formule

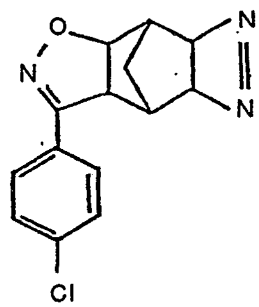

10. Composé de formule

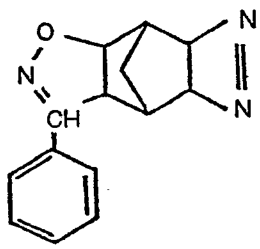

11. Composé de formule

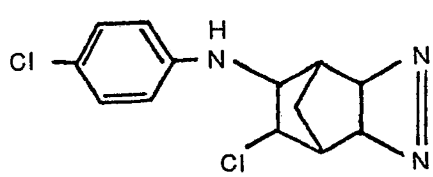

12. Moyen pour influencer la croissance des plantes, contenant un composé selon la revendication 1.

13. Moyen pour influencer la croissance des plantes, contenant un support solide ou liquide et un composé selon la revendication 1.

14. Procédé de préparation d'un moyen pour influencer la croissance des plantes caractérisé par le fait qu'on mélange un support solide ou liquide avec un composé selon la revendication 1.

15. Procédé pour influencer la croissance des plantes caractérisé par le fait qu'on traite les plantes ou leur biotope avec un composé selon la revendication 1.

## Claims

1. Heterocyclic norbornane derivatives of the formula

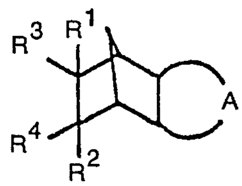

where A is 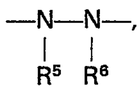, —N=N—, 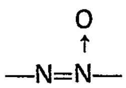 or 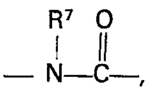,

$R^5$ and $R^6$ each being hydrogen or alkoxycarbonyl and $R^7$ being hydrogen or $—SO_2Cl$, and $R^1$ and $R^2$ together denote

—O—, 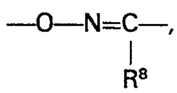, 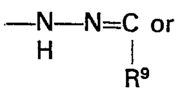 or 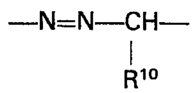

and, if A is 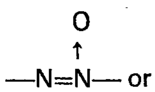 or 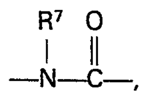, 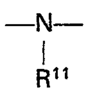 or 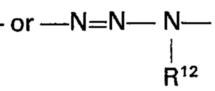

$R^8$ to $R^{12}$ each being phenyl optionally substituted by halogen, nitro, alkoxy, prim-, sec- or tert-butyl or haloalkyl, or 2-thiazolyl, or $R^1$ and $R^2$ as individual radicals are each halogen, or one of these radicals is halogen, OH or alkoxycarbonyl and the other radical is $R^{12}$ NH—, and $R^3$ and $R^4$ each denote hydrogen, one of these radicals also being halogen if $R^1$ and $R^2$ together denote —O—.

2. A compound of the formula

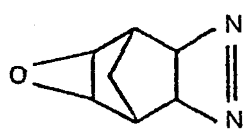

3. A compound of the formula

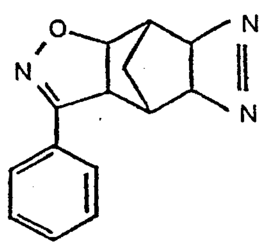

4. A compound of the formula

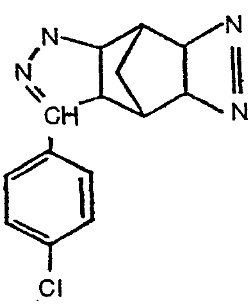

5. A compound of the formula

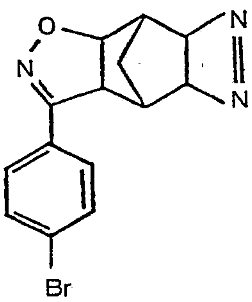

6. A compound of the formula

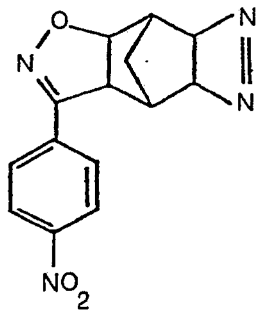

7. A compound of the formula

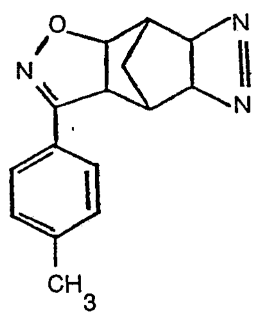

8. A compound of the formula

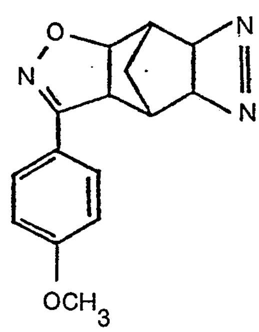

9. A compound of the formula

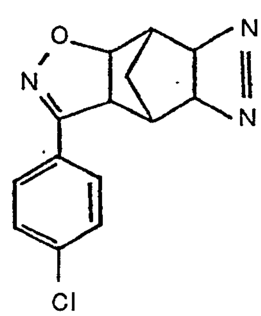

10. A compound of the formula 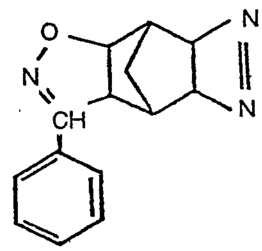

11. A compound of the formula 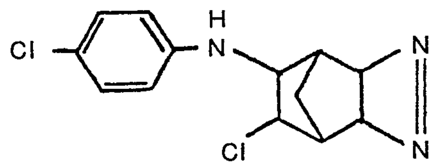

12. An agent for influencing plant growth containing a compound as claimed in claim 1.

13. An agent for influencing plant growth containing a solid or liquid carrier and a compound as claimed in claim 1.

14. A process for the production of an agent for influencing plant growth, characterized in that a solid or liquid carrier is mixed with a compound as claimed in claim 1.

15. A process for influencing plant growth, characterized in that the plant or its environment is treated with a compound as claimed in claim 1.